(19) **Europäisches Patentamt**
**European Patent Office**
**Office européen des brevets**

(11) **EP 2 917 367 B1**

(12) **EUROPEAN PATENT SPECIFICATION**

(45) Date of publication and mention of the grant of the patent:
**14.12.2016 Bulletin 2016/50**

(51) Int Cl.:
*C12Q 1/68* (2006.01)    *G06F 19/20* (2011.01)

(21) Application number: **13792613.5**

(22) Date of filing: **06.11.2013**

(86) International application number:
**PCT/EP2013/073100**

(87) International publication number:
**WO 2014/072309 (15.05.2014 Gazette 2014/20)**

(54) **A METHOD OF IMPROVING MICROARRAY PERFORMANCE BY STRAND ELIMINATION**

VERFAHREN ZUR VERBESSERUNG EINER MIKROARRAYLEISTUNG MITTELS ENTFERNUNG VON STRÄNGEN

PROCÉDÉ D'AMÉLIORATION DES PERFORMANCES DE PUCES À ADN PAR ÉLIMINATION DE BRIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2012 US 201261724156 P**

(43) Date of publication of application:
**16.09.2015 Bulletin 2015/38**

(73) Proprietors:
• **Roche Diagnostics GmbH**
**68305 Mannheim (DE)**
Designated Contracting States:
**DE**
• **F.Hoffmann-La Roche AG**
**4070 Basel (CH)**
Designated Contracting States:
**AL AT BE BG CH CY CZ DK EE ES FI FR GB GR HR HU IE IS IT LI LT LU LV MC MK MT NL NO PL PT RO RS SE SI SK SM TR**

(72) Inventor: **NAKAO, Aki**
**Sunnyvale, California 94087 (US)**

(74) Representative: **Poredda, Andreas**
**Roche Diagnostics GmbH**
**Patentabteilung**
**Sandhofer Strasse 116**
**68305 Mannheim (DE)**

(56) References cited:
**WO-A1-2011/040886**

• **LEE, CHARLIE WAH HENG ET AL: "Large-scale evolutionary surveillance of the 2009 H1N1 influenza A virus using resequencing arrays", NUCLEIC ACIDS RESEARCH, OXFORD UNIVERSITY PRESS, GB, vol. 38, no. 9, 24 February 2010 (2010-02-24), pages 1-14, XP008154979, ISSN: 0305-1048, DOI: 10.1093/NAR/GKQ089 [retrieved on 2010-02-24]**
• **ZHAN Y. ET AL: "Model-P: a basecalling method for resequencing microarrays of diploid samples", BIOINFORMATICS, vol. 21, no. Suppl 2, 1 September 2005 (2005-09-01), pages ii182-ii189, XP055053039, ISSN: 1367-4803, DOI: 10.1093/bioinformatics/bti1129**
• **PODDER MOHUA ET AL: "Dynamic variable selection in SNP genotype autocalling from APEX microarray data", BMC BIOINFORMATICS, BIOMED CENTRAL, LONDON, GB, vol. 7, no. 1, 30 November 2006 (2006-11-30), pages 521-1-521-11, XP021021661, ISSN: 1471-2105, DOI: 10.1186/1471-2105-7-521**
• **DIDION JOHN P. ET AL: "Discovery of novel variants in genotyping arrays improves genotype retention and reduces ascertainment bias", BMC GENOMICS, BIOMED CENTRAL LTD, LONDON, UK, vol. 13, no. 1, 19 January 2012 (2012-01-19), pages 34-1-34-18, XP021117835, ISSN: 1471-2164, DOI: 10.1186/1471-2164-13-34**

**EP 2 917 367 B1**

**(Cont. next page)**

- SEO JINWOOK ET AL: "Interactively optimizing signal-to-noise ratios in expression profiling: project-specific algorithm selection and detection p-value weighting in Affymetrix microarrays", BIOINFORMATICS, OXFORD UNIVERSITY PRESS, SURREY, GB, vol. 20, no. 16, 1 November 2004 (2004-11-01), pages 2534-2544, XP002368170, ISSN: 1367-4803, DOI: 10.1093/BIOINFORMATICS/BTH280 cited in the application
- MOLLA M ET AL: "A self-tuning method for one-chip SNP identification", COMPUTATIONAL SYSTEMS BIOINFORMATICS CONFERENCE, 2004. CSB 2004. PROCE EDINGS. 2004 IEEE STANFORD, CA, USA 16-19 AUG. 2004, PISCATAWAY, NJ, USA,IEEE, 16 August 2004 (2004-08-16), pages 66-76, XP010727067, DOI: 10.1109/CSB.2004.1332419 ISBN: 978-0-7695-2194-7
- TONYA LEBET ET AL: "Mutations causing severe combined immunodeficiency: detection with a custom resequencing microarray", GENETICS IN MEDICINE, vol. 10, no. 8, 1 August 2008 (2008-08-01), pages 575-585, XP055092989, ISSN: 1098-3600, DOI: 10.1097/GIM.0b013e31818063bc
- KARAMAN M W ET AL: "Comparisons of substitution, insertion and deletion probes for resequencing and mutational analysis using oligonucleotide microarrays", NUCLEIC ACIDS RESEARCH, OXFORD UNIVERSITY PRESS, GB, vol. 33, no. 3, 18 February 2005 (2005-02-18), pages 1-9, XP002368171, ISSN: 0305-1048, DOI: 10.1093/NAR/GNI034
- WONG CHRISTOPHER W ET AL: "Tracking the evolution of the SARS coronavirus using high-throughput, high-density resequencing arrays", GENOME RESEARCH, COLD SPRING HARBOR LABORATORY PRESS, WOODBURY, NY, US, vol. 14, no. 3, 1 March 2004 (2004-03-01), pages 398-405, XP002389740, ISSN: 1088-9051, DOI: 10.1101/GR.2141004
- DAVID J CUTLER ET AL: "High-Throughput Variation Detection and Genotyping Using Microarrays", GENOME RESEARCH, COLD SPRING HARBOR LABORATORY PRESS, WOODBURY, NY, US, vol. 11, no. 11, 1 January 2001 (2001-01-01), pages 1913-1925, XP008154971, ISSN: 1088-9051, DOI: 10.1101/GR.197201
- CRONIN M T ET AL: "Cystic fibrosis mutation detection by hybridization to light-generated DNA probe arrays", HUMAN MUTATION, JOHN WILEY & SONS, INC, US, vol. 7, no. 3, 1 January 1996 (1996-01-01), pages 244-255, XP008030268, ISSN: 1059-7794, DOI: 10.1002/(SICI)1098-1004(1996)7:3<244::AID-HUMU9>3.0.CO;2-A

**Description**

BACKGROUND OF THE INVENTION

Oligonucleotide microarrays (chips) are an economical way of analyzing multiple nucleic acid targets in one experiment. These arrays are commonly used to analyze multiple genes, for example in a gene expression assay. However, oligonucleotide microarrays are also gaining popularity as an economical and convenient alternative to sequencing in somatic and germline mutation detection assays. Certain genes are well known as somatic mutation and polymorphism hotspots. For such genes, many of the somatic mutations and polymorphisms are associated with a disease or an altered phenotype. For example, multiple mutations in the TP53 and EGFR genes are relevant to cancer. Somatic mutations in TP53 gene are known to cause loss of p53 function, associated with an increase of cancers occurring in various tissues. The TP53 mutation status is also useful for prognosis and predicting response to therapy. Similarly, multiple polymorphisms in CYP450 gene effectively predict the pattern of drug metabolism. Because of the large number of mutations, targeting each mutation with a separate assay becomes impractical. Thus microarrays capable of at once probing multiple mutated base positions (or even every base position within the gene) offer a useful alternative.

A typical microarray (chip) is a collection of microscopic spots each containing millions of nucleic acid probes attached to a solid surface. The probes are capable of hybridizing to the labeled DNA fragments from a sample under suitable conditions. Probe-target hybridization is detected and optionally quantified by detection of a label conjugated to the target molecule.

Microarrays as a mutation detection tool have been validated in several systems (reviewed in Schwartz, S., Clinical Utility of Single Nucleotide Polymorphism Arrays (2011) Clin. Lab. Med. 31:581.) Unfortunately, studies involving microarrays report that the sensitivity and specificity of microarrays are not yet ideal compared to existing technologies (*see e.g.* Zin R., et al., SNP-based arrays complement classic cytogenetics in the detection of chromosomal aberrations in Wilms' tumor (2012) Cancer Genetics 205:80).

Lee C.W.H., et al.,Large-scale evolutionary surveillance of the 2009 H1N1 influenza A virus using resequencing arrays(2010) Nucleic Acids Research 38(9):1-14, and corresponding patent application WO2011040886A1, disclose elimination of the bad strand in a basecall of a microarray test sample if different performance is detected from the two strands at a particular position.

Zhan Y. and Kulp D., Model-P: A basecalling method for resqeuencing microarrays of diploid samples(2005) Bioinformatics 21 Suppl.2, p.ii182-ii189 discloses a basecalling method for resequencing microarrays of diploid samples, wherein the final genotype call is determined by the strand with the higher confidence score.

In practice, it appears that performance of microarrays is not uniform throughout the probed sequence. Some positions within the sequence are subject to error more than others. The use of better mathematical or statistical tools for data analysis that identify such special sites holds the promise of improving sensitivity and specificity of mutation detection microarrays.

SUMMARY OF THE INVENTION

In some embodiments, the invention is a method of interrogating a sequence of a target nucleic acid having a sense and an anti-sense strand by a microarray analysis comprising a sequence determination computation, the method comprising omitting from the computation a predetermined signal from one of the sense and anti-sense strands for one or more nucleotide positions in the target nucleic acid sequence determined by analysis of one or more training samples, wherein omitting the signal from one of the sense and anti-sense strands at a nucleotide position comprises the steps of: using one or more training samples and a plurality of microarrays, comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands; for each probe set, determining base discrimination ability by comparing the hybridization signals within each probe set; for each nucleotide position, computing discrimination ability for sense and antisense strand separately using the computed discrimination ability from each of the probe sets; for each nucleotide position, comparing the computed discrimination ability between the sense and the anti-sense strands; in the test sample, omitting the signal from the strand with lower base discrimination ability using the one or more training samples. In certain embodiments, the method further comprises determining whether the difference in base discrimination falls above a threshold value and omitting the signal from a strand corresponding to probe sets with lower base discrimination if the difference falls above the threshold value. In some embodiments, the probe intensities are measured using a plurality of microarrays contacted with a single sample. In some embodiments, the hybridization signal is measured using a plurality of microarrays contacted with a plurality of samples. In variations of this embodiment, the base discrimination is measured using Formula 1. In further variations of this embodiment, the discrimination ability for sense and antisense strand is computed as a percentile of the discrimination ability for probe sets in the strand at the base position. In yet further variations of this embodiment, the discrimination ability between sense and antisense strand is compared using Formula 3. In certain embodiments the percentile is between 60 and 90%. In other embodiments, the percentile is the third quartile (75%). In other embodiments, the invention is a method of detecting the presence or absence of a target nucleic acid having a sense and an anti-sense strands in a test sample using a microarray analysis including a sequence determination or mutation detection computation, comprising omitting from the computation a predetermined signal from one of the sense and anti-sense strands for one or more nucleotide positions in the target nucleic acid sequence determined by analysis of one or more training samples, wherein omitting the signal from one of the sense and anti-sense strands at a nucleotide position comprises the steps of: using one or more training samples and a plurality of microarrays, comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands; for each probe set, determining base discrimination ability by comparing the hybridization signals within each probe set; for each nucleotide position, computing discrimination ability for sense and antisense strand separately using discrimination ability from each of the probe sets; for each nucleotide position, comparing discrimination ability between the sense and the anti-sense strands; in the test sample omitting the signal from a strand with lower base discrimination ability using the one or more training samples. In certain embodiments the method further comprises determining whether the difference in base discrimination falls above a threshold value and omitting the signal from a strand corresponding to probe sets with lower base discrimination if the difference falls above the threshold value. In variations of this embodiment, the base discrimination is measured using Formula 1. In further variations of this embodiment, the discrimination ability of the sense and anti-sense strand is computed as a percentile of the discrimination ability for probe sets in the strand at the base position measured using a plurality of microarrays.

Also described herein is a computer readable medium including code for controlling one or more processors to detect the presence or absence of a target nucleic acid having a sense and an anti-sense strands in a test sample using a microarray analysis that includes a sequence determination or mutation detection computation, comprising omitting from the computation a signal from one of the sense and anti-sense strands for one or more nucleotide positions in the target nucleic acid sequence. In variations of this embodiment, the computer readable medium comprises a code controlling the steps of: using a plurality of microarrays, measuring hybridization signals at the nucleotide position using one or more probe sets for each of the sense and the anti-sense strands; for each probe set, determining base discrimination ability by comparing the hybridization signals within each probe set; for each nucleotide position, computing discrimination ability for sense and antisense strand separately using discrimination ability from each of the probe sets; for each nucleotide position, comparing discrimination ability between the sense and the anti-sense strands; omitting the signal from a strand with lower base discrimination ability.

In yet another embodiment, the invention is a system for detecting a target nucleic acid in a test sample comprising: a data acquisition module configured to acquire hybridization data from a microarray; a data processing unit configured to process the data to determine a target nucleotide sequence by omitting the predetermined signal from one of the sense and anti-sense strands at one or more nucleotide positions in the target sequence determined by analysis of one or more training samples via the steps of: using one or more training samples and a plurality of microarrays, comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands; for each probe set, determining base discrimination ability by comparing the hybridization signals within each probe set; for each nucleotide position, computing discrimination ability for sense and antisense strand separately using discrimination ability from each of the probe sets; for each nucleotide position, comparing discrimination ability between the sense and the anti-sense strands; in the test sample omitting the signal from a strand with lower base discrimination ability determined using the one or more training samples; and a display module configured to display the data produced by the data processing unit. In certain embodiments comparing the base discrimination between the probe sets corresponding to the sense and the anti-sense strands is performed by comparing a percentile of the base discrimination from sense and antisense strands obtained from the plurality of microarrays.

A further aspect of the present disclosure is a method of detecting the presence or absence of a mutation in the p53 gene in a test sample using a microarray analysis including a mutation detection computation, comprising omitting from the computation a signal from one of the sense and anti-sense strands for one or more nucleotide positions in the target nucleic acid sequence. In variations of this embodiment, the nucleotide positions are selected from codon 273, position 1 and codon 220, position 2 within the p53 gene.

BRIEF DESCRIPTION OF THE FIGURES

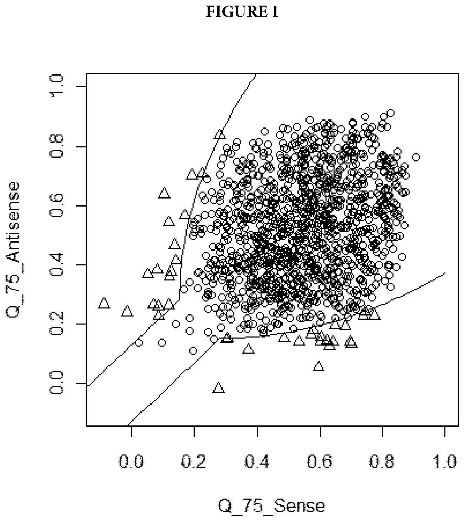

Figure 1: Selecting strands for elimination by comparing discriminating ability of sense and antisense probes using $Q_{75}$ value.

DETAILED DESCRIPTION OF THE INVENTION

*Definitions*

The terms "nucleic acid," and "oligonucleotide" refer to target sequences and probes. The terms are not limited by length and are generic to linear polymers of deoxyribonucleotides (single-stranded or double-stranded DNA), ribonucleotides (RNA), and any other N-glycoside of a purine or pyrimidine base, including adenosine, guanosine, cytidine, thymidine and uridine and modifications of these bases.

The term "probe" refers to an oligonucleotide that selectively hybridizes to a target nucleic acid under suitable conditions.

The term "probe set" refers to a group of two or more probes in a microarray designed to interrogate the mutation status in the same base position within a target sequence. A typical probe set contains five or more different probes; one for hybridizing to wildtype DNA sequence from a sample, three probes for three possible single-base substitutions, and one probe for detecting a single base pair deletion at the interrogating position. Additional probes may be added, e.g. the sixth probe can be included to detect two base-pair deletion.

The term "target site" or "target base position" refers to the base position in the target nucleic acid that is interrogated by a probe in the probe set. Multiple overlapping probes within the probe sets may interrogate the same target site.

The terms "target sequence" or "target" refer to a region of a nucleic acid sequence that is to be analyzed.

The term "sample" refers to any composition containing or presumed to contain nucleic acid. This includes a sample of tissue or fluid isolated from an individual for example, skin, plasma, serum, spinal fluid, lymph fluid, synovial fluid, urine, tears, blood cells, organs, bone marrow and tumors, including the fresh or fresh-frozen tissue and formalin-fixed paraffin embedded tissue (FFPET), and also to samples of *in vitro* cultures established from cells taken from an individual, and nucleic acids isolated therefrom.

The term "training set" refers to a set of samples used to build data analysis algorithms including statistical models.

The term "training data set" refers to a set of microarray data obtained from the training set. A training data set may be a set of microarray data obtained from samples where the sequences are known. For example, the training data set may be used to build a statistical model to determine the mutation status and to identify specific nucleotide positions where the intensity patterns are consistently different from the rest within the sequence and modify the mutation detection algorithm.

The terms "testing set" refers to a set of one or more samples used to verify the mutation detection algorithm built using the training set.

The term "testing data set" refers to a set of microarray data obtained from the testing set. A testing data set may be a set of microarray data obtained from samples where the sequences (mutation status) are known. For example, the testing data set may be used to verify effective mutation detection by the algorithm built based on the data from the training data set.

The term "test sample" refers to a sample used to generate testing dataset.

The term "re-sequencing by microarray" or "mutation detection by microarray" are used interchangeably to refer to a method of mutation detection within the target sequence by detecting and analyzing hybridization signals from multiple probe sets arranged on a microarray, each set corresponding to a nucleotide position within the sense and the anti-sense strand of the target sequence, hybridized to the labeled nucleic acid fragments present in a sample. Typically, re-sequencing by microarray comprises an algorithm that detects mutated nucleic acid in a background of wild-type nucleic acid utilizing the hybridization signals from multiple probe sets. The term "re-sequencing" encompasses determination of mutation status in the entire sequence of the target nucleic acid as well as determination of fewer than all nucleotides, e.g. only one or several selected nucleotides in the target nucleic acid that are known sites of mutations.

The present invention comprises a method of improving the accuracy of re-sequencing and mutation detection microarrays. A microarray is a collection of nucleic acid probes designed to detect mutations in a background of wild-type nucleic acid sequences. That is, under suitable hybridization conditions, the probes would preferentially hybridize only to the target sequence present in the sample genome. Each probe set is designed to detect three possible single base pair substitutions and a single base pair deletion for a particular nucleotide position within the target sequence. Several overlapping probe sets with different probe length may be designed to detect an individual mutation. A microarray may contain probe sets designed to detect mutations in some or all of the nucleotides in the target sequence. Furthermore, a microarray may contain probe sets corresponding to nucleotides on both strands of the target sequence. Depending on the number of nucleotides to be interrogated, an array may contain thousands or even millions of probe sets (*see* Schena, M. (ed.), Microarray Biochip Technology (2000) Eaton Pub. Co. (Westborough, Mass.)).

Each probe set typically contains five probes: four for each of the possible nucleotides at the particular position within the target sequence and one probe for a deletion of the nucleotide at that position. Upon incubation under appropriate protocols, the probes emit detectable signals. Ideally, one of the five probes within the probeset emits a much greater signal than the other four if the interrogating position is wild-type, and two of the five probes emit greater signals than the other three when the sample contains mutated DNA in addition to the wild-type DNA. Since most of the somatic mutations are heterozygous, and a typical clinical sample contains both cancer and non-cancer cells, wild-type signals are present for most of the cases. The detector registers the signal associated with a particular probe for a particular strand for a particular nucleotide at that position within the target sequence.

The software algorithm currently used for making nucleotide calls, examines the data from both sense and antisense probe sets for each position. Only when both sense and anti-sense signals are in a certain agreement on a mutation in a particular position within the target sequence, the software makes the mutation call for the nucleotide at that position.

The present invention is a method of improving mutation detection or re-sequencing by microarray analysis comprising omitting a signal from one of the two complementary strands from a mutation detection algorithm for one or more nucleotide positions in the target sequence.

The inventors observed that each array trends towards a number of trouble spots within the target sequence. These trouble spots become apparent when the array is tested with multiple samples of different origin and different quality of nucleic acids to be tested. The array is consistently unable to make correct calls for certain positions within the target sequence. Aiming to eliminate or reduce the number of such missed calls, the inventors investigated the source of error at the trouble spots. Surprisingly, it was discovered that for some positions within the target sequence, there was a dramatic difference in performance between the sense and antisense probe sets. Accordingly, the inventors devised a mathematical method to identify such nucleotide positions and eliminate the data obtained from the poorly performing strand from computation. The strands with poor performance are identified according to the teaching of the invention. It is noted that where both sense and antisense probes perform poorly; probes from neither strand are eliminated.

In one embodiment, the invention is a method comprising obtaining a microarray data set by hybridizing the labeled and fragmented nucleic acids from a sample to an oligonucleotide microarray and obtaining the hybridization data and converting the hybridization data into the probe intensities and analyzing the probe intensity data to extract biologically meaningful information such as nucleic acid sequence or presence of mutations. Oligonucleotide microarrays may be custom made as described in Schena, M. (ed.), Microarray Biochip Technology (2000) or obtained from commercial suppliers such as *e.g.,* Affymetrix (Santa Clara, Cal.), NimbleGen (Madison, Wisc.), and Agilent Tech. (Santa Clara, Cal.). The optimal conditions for generating high quality microarray data, such as sample preparation, amplification, fragmentation and labeling of nucleic acids, hybridization and washing may be obtained from the manufacturers of microarrays, or determined empirically by one skilled in the art of nucleic acid chemistry. To determine the sample sequence or identify mutations in a sample, the microarray hybridization data may be analyzed by any microarray analysis algorithm known in the art, e.g., Microarray Suite (MAS), or Gene Chip Operating System (GCOS) (Affymetrix, Santa Clara, Cal.).

In this embodiment, multiple microarray experiments are conducted to obtain microarray data sets and identify persistent trouble spots within the target nucleic acid sequence. Optionally multiple experiments are conducted with target nucleic acids isolated from various sources to identify trouble spots that are independent of the source and quality of the nucleic acid in the sample. In the embodiments of the invention, prior to analyzing a test sample, one or more training samples are analyzed to identify trouble spots in the training data sets. The training samples may comprise mixtures of target nucleic acids with and without mutations to simulate patient samples that contain mutant and non-mutant cells.

In some embodiments of the invention, the trouble spots in the microarray data sets are further analyzed to determine whether the probe sets targeting one strand or both strands consistently fail to make the call. In variations of this embodiment, the microarray data sets are analyzed to determine whether the probe sets failing to make the mutation call at the trouble spot are prone to non-specific hybridization.

In some embodiments, the invention comprises a method of identifying poorly performing strands to be eliminated from the sequence determination or mutation detection computation at certain nucleotide positions within the target sequence. To identify whether one strand should be eliminated at a nucleotide position, the following steps may be performed using probe hybridization data for the nucleotide position:

(1) calculate discriminating ability of each probe set;
(2) calculate a percentile of discriminating ability of all probe sets for sense and antisense strands separately for each of the target nucleotide positions;

(3) determine a desired percentile of the values obtained in step (2) across multiple microarrays;

(4) determine the difference between the discriminating ability at the given percentiles obtained in step (3) between the sense and the anti-sense strands;

(5) where the difference obtained in step (4) is substantial or falls above a threshold, eliminate from the mutation detection or re-sequencing computation the strand with the lower discriminating ability.

Any formula for calculating discriminating ability of a probe set may be used. Seo, et. al, Bioinformatics, Vol.20, No. 16 2534-2544, 2004). In some embodiments, the discrimination ability may be determined by calculating discrimination ratio of mismatch probe (DR_MM). DR_MM for each probe set (DR_MM$_s$) is determined according to Formula 1:

$$DR\_MM_s = \{(PM - \max(MM_i)) / (PM + \max(MM_i)), i = 1{:}3\} \qquad \text{Formula 1}$$

PM - probe intensity of the perfectly matched probe, which is designed to hybridize to the wild-type sequence for the base position

$MM_i$ - probe intensity of one of the three mismatched probe, which is designed to hybridize to a single base pair substitution

max(MM) - the maximum probe intensity among the three mismatched probes in the probe set

Any percentile may be used to compute discriminating ability of a strand at a base position using the DR_MM calculated for each probes set, for example, 50th (median), 55th, 60th, 70th, 75th (quartile), 80th or 90th percentiles or any percentiles falling between those numbers. In some embodiments, 50th (median) is used.

Any percentile may be used to identify a poorly performing strand based on the performance of a strand at a base position across multiple microarrays, for example, 55th, 60th, 70th, 80th or 90th percentiles may be used or any percentile falling between those numbers. In some embodiments, the 75th percentile ($Q_{75}$) is used.

Any threshold for the difference between the percentiles of the sense and anti-sense strands may be used for strand elimination. In some embodiments, the threshold is set according to Formula 2:

$$Q_{75i} < Q_{75j} - T \qquad \text{Formula 2}$$

T is the threshold

$Q_{75i}$ is the $Q_{75}$ value of a strand i to be eliminated;

$Q_{75j}$ is the $Q_{75}$ value of the complementary strand j.

In some embodiments, the threshold equals 0.13 (T=0.13).

In some embodiments, the chosen value of the threshold depends on relative performance of the strands. When discriminating ability of one strand is poor and the difference with the other strand exceed the threshold, the poor strand is eliminated. When discriminating ability is moderately poor, the strand is eliminated only if the other strand performs significantly better, *i.e.* $Q_{75}$ is substantially higher than the threshold. If both strands perform poorly, no strand is eliminated. In some embodiments, extremely poor performance is defined as $Q_{75}<0.151$ and moderately poor performance is defined as $0.151 \leq Q_{75} < 0.3$. A threshold value for poor performance (PT) may be empirically determined.

In some embodiments, the threshold may be set according to Formula 3:

$$(1) \quad Q_{75i} < Q_{75j} - T, \qquad\qquad\qquad \text{for } Q_{75i} < PT$$

$$(2) \quad Q_{75i} < A(Q_{75j} - B)^2 + PT, \qquad\qquad \text{for } Q_{75i} \geq PT \qquad \text{Formula 3}$$

T is the threshold for the difference between the strands;

PT is the threshold for poor performance

A and B are empirically determined

$Q_{75i}$ is the $Q_{75}$ value of a strand i to be eliminated;

$Q_{75j}$ is the $Q_{75}$ value of the complementary strand j.

In some embodiments, the threshold is 0.13 (T=0.13), the threshold for poor performance is 0.151 (PT= 0.151), A=0.42227 and B=0.281.

In some embodiments, the invention comprises a data analysis algorithm for microarrays that are designed to detect mutations in a target nucleotide sequence by omitting the signal from one of the sense and anti-sense strands at one or more nucleotide positions in the target sequence via the steps of: using multiple microarrays, measuring hybridization signals (e.g., probe intensities) at the nucleotide position using one or more probe sets for each of the sense and the anti-sense strands; for each probe set, computing base discrimination ability within each probe set; calculating a percentile of discriminating ability of all probe sets for sense and antisense strands separately for each of the target nucleotide positions; determining a desired percentile of the discriminating ability values across multiple microarrays; determining the difference between the discriminating ability at the given percentiles between the sense and the anti-sense strands; where the difference is substantial or falls above a threshold, eliminating from the mutation detection or re-sequencing computation the strand with the lower discriminating ability.

In some embodiments, the invention comprises a data analysis algorithm for microarrays that are designed to detect mutations in the TP53 gene in a sample by omitting the signal from one of the sense and anti-sense strands at one or more nucleotide positions in the target sequence via the steps of: using multiple microarrays, measuring hybridization signals (e.g., probe intensities) at the nucleotide position using one or more probe sets for each of the sense and the anti-sense strands; for each probe set, computing base discrimination ability using the signals within each probe set; calculating a percentile of discriminating ability of all probe sets for sense and antisense strands separately for each of the target nucleotide positions; determining a desired percentile of the discriminating ability values across multiple microarrays; determining the difference between the discriminating ability at the given percentiles between the sense and the anti-sense strands; where the difference is substantial or falls above a threshold, eliminating from the mutation detection or re-sequencing computation the strand with the lower discriminating ability. These mutations are associated with development and progression of certain human cancers. *See e.g.* Freed-Pastor, W. et al. (2004). "Mutant p53: one name, many proteins" (2012) Genes Dev. 26:1268.

Examples and figures below illustrate applications of the method of the present invention to detecting mutations in the human TP53 (p53) gene.

In some embodiments, the invention is a computer readable medium including code for controlling one or more processors to determine a target nucleotide sequence by omitting the signal from one of the sense and anti-sense strands at one or more nucleotide positions in the target sequence via the steps of: using multiple microarrays, measuring hybridization signals (e.g., probe intensities) at the nucleotide position using one or more probe sets for each of the sense and the anti-sense strands; computing base discrimination ability using the signals within each probe set; calculating a percentile of discriminating ability of all probe sets for sense and antisense strands separately for each of the target nucleotide positions; determining a desired percentile of the discriminating ability values across multiple microarrays; determining the difference between the discriminating ability at the given percentiles between the sense and the anti-sense strands; where the difference is substantial or falls above a threshold, eliminating from the mutation detection or re-sequencing computation the strand with the lower discriminating ability.

In some embodiments, the invention is a system for determining a target nucleotide sequence in a test sample according to the present invention comprising: a data acquisition module configured to acquire a data set from nucleotide microarrays, the data set containing data from the sense and the anti-sense strands; a data processing unit configured to process the data to determine a target nucleotide sequence by omitting the signal from one of the sense and anti-sense strands at one or more nucleotide positions in the target sequence via the steps of: using multiple microarrays, measuring hybridization signals (*e.g.,* probe intensities) at the nucleotide position using one or more probe sets for each of the sense and the anti-sense strands; for each probe set, computing base discrimination ability within the probe set; calculating a percentile of discriminating ability of all probe sets for sense and antisense strands separately for each of the target nucleotide positions; determining a desired percentile of the discriminating ability values across multiple microarrays; determining the difference between the discriminating ability at the given percentiles between the sense and the anti-sense strands; where the difference is substantial or falls above a threshold, eliminating from the mutation detection or re-sequencing computation the strand with the lower discriminating ability; and a display module configured to display the data produced by the data processing unit.

EXAMPLES

***Example 1. Identifying trouble spots within the p53 gene in AMPLICHIP® p53.***

Feasibility studies of AMPLICHIP® p53 (Roche Molecular Diagnostics, Indianapolis, Ind.) to detect mutations in the p53 gene (TP53) showed that detection ability of the following two mutations is not satisfactory: (1) 220_2 (codon 220, 2nd position) A>G, and (2) 273_1 (codon 273, 1st position) C>T. These are among the top six most prevalent mutations found in ovarian cancer.

To confirm the trouble spots, a training set of samples simulating patient samples were prepared as described in Table 1. The samples consisted of mixtures of mutant and wild-type DNA. Mutant DNA was obtained from a cell line whose mutant status at codon 273 was confirmed prior to the study. The mutant DNA was diluted with a p53-wild-type cell line DNA.

*Table 1. Samples used to evaluate 273_1 C>T mutation detection ability*

| Sample | Codon | Base change | % mutant DNA |
|---|---|---|---|
| 1 | 273_1 | C>T | 20 |
| 1 | 273_1 | C>T | 25 |
| 1 | 273_1 | C>T | 33 |
| 2 | 273_1 | C>T | 20 |
| 2 | 273_1 | C>T | 25 |
| 2 | 273_1 | C>T | 33 |

***Example 2. Investigation of the nature of the trouble spots.***

A series of analyses of microarray data revealed the following trends for all chips that failed to call the A>G mutation at codon 220_2:

1) The probe set targeting the sense strand calls the A>G mutation but the antisense probe set calls wild type;
2) High amount of cross-hybridization for all probes is observed for the antisense probe sets;
3) *In silico* analysis of probes in the antisense probe set supports high amount of cross-hybridization.

A series of analyses of microarray data using cell line and clinical samples revealed the following trends for all chips that failed to call the C>T mutation at codon 273_1:

1) The probe set targeting the sense strand calls the C>T mutation but the antisense probe set calls wild type;

2) High amount of cross-hybridization for some probes is observed for the antisense probe set.

***Example 3. Algorithm modification by strand elimination***

A total of 123 reference chips (microarrays) were used (AMPLICHIP® p53 (Roche Molecular Diagnostics, Indianapolis, Ind.)). A reference chip is hybridized with a wild-type cell line and the probe intensities are is used as a baseline to detect mutation in a clinical sample. First, the median of DR_MM (discrimination ratio of mismatch probes) was calculated for each probe set in a base position on each strand for each chip according to Formula 1. DR_MM is a good measure to evaluate the amount of non-specific hybridization. Then, the 75th percentile value ($Q_{75}$) across the 123 chips was calculated per base position per strand. A very low $Q_{75}$ value means that the discrimination ability of the probe sets on the strand of the base position is quite poor 75% of the time. Using the $Q_{75}$ values for sense and antisense strands at the same nucleotide position, the strands were eliminated according to Formula 3 with the following values:

$$(1) \qquad Q_{75i} < Q_{75j} - 0.130, \qquad\qquad \text{for } Q_{75i} < 0.151$$

$$(2) \qquad Q_{75i} < 0.4227(Q_{75j} - 0.281)^2 + 0.151, \qquad \text{for } Q_{75i} \geq 0.151$$

$Q_{75i}$ is the $Q_{75}$ value of a strand i to be eliminated;
$Q_{75j}$ is the $Q_{75}$ value of the complementary strand j

A total of 39 strands were identified and eliminated, *i.e.* not used for making the mutation calls. They are shown as triangle data points in Figure 1 and summarized in Table 1.

Figure 1 illustrates comparison of discriminating ability of sense and antisense probes using $Q_{75}$ value calculated using 123 chips hybridized with p53 wild-type cell line DNA. The axes represent the $Q_{75}$ value for each sense and anti-sense strands. Each data point represents one of the 1240 interrogated base positions. The two lines enclose the data points for which no strand is eliminated (circle data points in Figure 1). The data points fall within three general categories. In the first category, (most cases) the $Q_{75}$ values for the sense and the anti-sense strands are comparable. These cases fall within the enclosure and not far from the diagonal ($Q_{75}$_Antisense = $Q_{75}$_Sense) line on Figure 1. In the second category, the data points have different $Q_{75}$ values between sense and anti-sense strands but both values are still reasonably high, (*e.g.,* above 0.2), representing acceptable discrimination ability for the corresponding base position. These data points also fall within the enclosure on Figure 1. In the third category, the $Q_{75}$ values are quite different between sense and antisense strands, representing large difference in discriminating ability between the strands, and one strand has quite low $Q_{75}$, indicating poor discriminating ability. These data points fall outside the enclosure on Figure 1. For the base positions corresponding to data points within the third category, the worse preforming strand (lower $Q_{75}$) is eliminated from the mutation calling computation.

In Table 2, "Codon" indicates the codon number and position of the nucleotide within the codon; "Wt" indicates the nucleotide in the wild type sequence; "S" indicates sense strands and "AS" indicates anti-sense strands; "Q75_S" and "Q75_AS" indicate $Q_{75}$ for the sense and the anti-sense strand respectively; and "abs(Diff75)" indicates the absolute value of the differences between the sense and the anti-sense strands.

*Table 1. Selecting strands for elimination from mutation calling computation*

| Codon | Wt | Q75_S | Q75_AS | abs(Diff75) | Strand To Eliminate |
|-------|----|-------|--------|-------------|---------------------|
| 138_1 | G | 0.0808 | 0.3808 | 0.3000 | S |
| 144_3 | G | 0.1902 | 0.7001 | 0.5099 | S |
| 151_2 | C | 0.0827 | 0.2584 | 0.1757 | S |
| 151_3 | C | 0.0691 | 0.2647 | 0.1956 | S |
| 152_1 | C | -0.0894 | 0.2665 | 0.3559 | S |
| 157_1 | G | 0.1184 | 0.2616 | 0.1433 | S |
| 158_2 | G | 0.1409 | 0.4131 | 0.2722 | S |
| 159_1 | G | 0.0496 | 0.3657 | 0.3162 | S |
| 160_3 | G | 0.0857 | 0.2257 | 0.1401 | S |
| 161_1 | G | -0.0155 | 0.2378 | 0.2532 | S |
| 180_3 | G | 0.1682 | 0.5666 | 0.3984 | S |
| 226_2 | G | 0.2802 | 0.8360 | 0.5558 | S |
| 248_3 | G | 0.2241 | 0.7074 | 0.4834 | S |
| 249_1 | A | 0.1243 | 0.3757 | 0.2514 | S |
| 249_3 | G | 0.1164 | 0.5409 | 0.4245 | S |
| 282_3 | G | 0.1350 | 0.4658 | 0.3308 | S |
| 336_3 | G | 0.1194 | 0.3569 | 0.2375 | S |
| 347_1 | G | 0.1017 | 0.6385 | 0.5368 | S |
| 12_1 | C | 0.5775 | 0.1767 | 0.4008 | AS |
| 72_3 | C | 0.6312 | 0.1219 | 0.5093 | AS |
| 74_2 | C | 0.5724 | 0.1617 | 0.4108 | AS |
| 110_3 | T | 0.6019 | 0.1390 | 0.4630 | AS |

(continued)

| Codon | Wt | Q75_S | Q75_AS | abs(Diff75) | Strand To Eliminate |
|-------|-----|--------|---------|-------------|---------------------|
| 138_2 | C | 0.6415 | 0.1386 | 0.5029 | AS |
| 150_1 | A | 0.6215 | 0.1428 | 0.4787 | AS |
| 158_3 | C | 0.5340 | 0.1379 | 0.3961 | AS |
| 161_2 | C | 0.5952 | 0.0528 | 0.5424 | AS |
| 175_1 | C | 0.6036 | 0.1544 | 0.4492 | AS |
| 220_1 | T | 0.3029 | 0.1460 | 0.1569 | AS |
| 220_2 | A | 0.3063 | 0.1507 | 0.1557 | AS |
| 273_1 | C | 0.7451 | 0.2410 | 0.5041 | AS |
| 274_2 | T | 0.4855 | 0.1507 | 0.3348 | AS |
| 276_3 | C | 0.7741 | 0.2275 | 0.5466 | AS |
| 299_1 | C | 0.6799 | 0.1919 | 0.4880 | AS |
| 318_3 | A | 0.7385 | 0.2273 | 0.5112 | AS |
| 337_1 | C | 0.2766 | -0.0205 | 0.2971 | AS |
| 337_3 | C | 0.3730 | 0.1116 | 0.2614 | AS |
| 347_2 | C | 0.6472 | 0.1949 | 0.4523 | AS |
| 355_2 | C | 0.7003 | 0.1325 | 0.5678 | AS |
| 390_1 | C | 0.6971 | 0.1379 | 0.5592 | AS |

Eliminating the strands from the mutation detection computation resulted in improved mutation detection ability of a re-sequencing microarray (AMPLICHIP® p53 Test) without compromising specificity. Examples of some datasets are shown in Table 3.

Table 3. Mutation detection ability of the AMPLICHIP® Microarray with and without strand elimination

| Dataset / Study Name | Algorithm Ver 4.0.1 Mutations detected without strand elimination | | Algorithm Ver 4.0.4 Mutations detected with strand elimination | |
|----------------------|--------|---------|--------|-----------|
| Dataset 1, clinical samples (n=51) | 94.3% | (33[b]/35[a]) | 97.1% | (34/35) |
| Dataset 2, clinical samples (n=60) | 90.9% | (30/33) | 97.0% | (32/33) |
| Clinical samples and cell lines (n=113) | 96.5% | (109/113) | 100.0% | (113/113) |
| Cell line containing 273_1 C>T with low 20-33mt% (n=12) | 0.0% | (0/12) | 100.0% | (12/12) |
| Algorithm test dataset, tumor >=50%, clinical samples (n=184) | 89.6% | (63/67) | 94.0% | (63/67) |
| Clinical samples, FFPE[c] and FF[d] (n=152) | 91.1% | (144/158) | 94.3% | (149/158) |
| [a]number of mutations already confirmed prior to this study [b]number of mutations AMPLICHIP® p53 correctly called out [c] Formalin-Fixed Paraffin Embedded [d] Fresh Frozen | | | | |

While the invention has been described in detail with reference to specific examples, it will be apparent to one skilled in the art that various modifications can be made within the scope of this invention. Thus the scope of the invention should not be limited by the examples described herein, but by the claims presented below.

**Claims**

1. A method of interrogating a sequence of a target nucleic acid having a sense and an anti-sense strand by a microarray analysis comprising a sequence determination computation, the method comprising omitting from the computation a predetermined signal from one of the sense and anti-sense strands for one or more nucleotide positions in the target nucleic acid sequence determined by analysis of one or more training samples, wherein omitting the predetermined signal from one of the sense and anti-sense strands at a nucleotide position comprises the steps of:

   a. using one or more training samples and a plurality of microarrays comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands;
   b. for each probe set, determining base discrimination ability by comparing the hybridization signals within each probe set;
   c. for each nucleotide position, computing discrimination ability for sense and antisense strand separately using discrimination ability from each of the probe sets determined in step (b);
   d. for each nucleotide position, comparing discrimination ability between the sense and the anti-sense strands computed in step (c);
   e. in the test sample omitting the signal from the strand with lower base discrimination ability identified in step (d) using the one or more training samples.

2. The method of claim 1, further comprising, in step (d), determining whether the difference in base discrimination falls above a threshold value and in step (e), omitting the signal from a strand corresponding to probe sets with lower base discrimination if the difference falls above the threshold value.

3. The method of any one of claims 1 or 2, wherein the probe intensities in step (a) are measured using a plurality of microarrays contacted with a single sample.

4. The method of any one of claims 1 or 2, wherein the hybridization signal in step (a) is measured using a plurality of microarrays contacted with a plurality of samples.

5. The method of any one of claims 1 to 4, wherein the base discrimination in step (b) is measured using Formula 1:

$$DR\_MM_s = \{(PM - \max(MM_i)) / (PM + \max(MM_i)), i = 1{:}3\},$$

wherein PM is the probe intensity of the perfectly matched probe, which is designed to hybridize to the wild-type sequence for the base position; MMi is the probe intensity of one of the three mismatched probe, which is designed to hybridize to a single base pair substitution; and max(MM) is the maximum probe intensity among the three mismatched probes in the probe set.

6. The method of any one of claims 1 to 5, wherein in step (c), discrimination ability for sense and antisense strand is computed as a percentile of the values obtained in step (b) for probe sets in the strand at the base position.

7. The method of any one of claims 1 to 6, wherein in step (d), discrimination ability between sense and antisense computed in step (c) is compared using Formula 3:

$$(1) \quad Q_{75i} < Q_{75j} - T, \qquad\qquad \text{for } Q_{75i} < PT$$

$$(2) \quad Q_{75i} < A(Q_{75j} - B)^2 + PT, \qquad for\ Q_{75i} \geq PT,$$

wherein T is the threshold; $Q_{75i}$ is the $Q_{75}$ value of a strand i to be eliminated; and $Q_{75j}$ is the $Q_{75}$ value of the complementary strand j.

8. The method of any one of claims 6 or 7, wherein the percentile is between 60 and 90%.

9. The method of any one of claims 6 to 8, wherein the percentile is the third quartile (75%).

10. A method of detecting the presence or absence of a target nucleic acid having a sense and an anti-sense strand in a test sample using a microarray analysis including a sequence determination or mutation detection computation, comprising omitting from the computation a predetermined signal from one of the sense and anti-sense strands for one or more nucleotide positions in the target nucleic acid sequence determined by analysis of one or more training samples, wherein omitting the predetermined signal from one of the sense and anti-sense strands at a nucleotide position comprises the steps of:

a. using one or more training samples and a plurality of microarrays comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands;
b. for each probe set, determining base discrimination ability by comparing the hybridization signals within each probe set;
c. for each nucleotide position, computing discrimination ability for sense and antisense strand separately using discrimination ability from each of the probe sets determined in step (b);
d. for each nucleotide position, comparing discrimination ability between the sense and the anti-sense strands computed in step (c);
e. in the test sample omitting the signal from a strand with lower base discrimination ability identified in step (d) using the one or more training samples.

11. The method of claim 10, further comprising, in step (d), determining whether the difference in base discrimination falls above a threshold value and in step (e), omitting the signal from a strand corresponding to probe sets with lower base discrimination if the difference falls above the threshold value.

12. The method of any one of claims 10 or 11, wherein the base discrimination ability in step (b) is measured using Formula 1:

$$DR\_MM_s = \{(PM - max(MM_i)) / (PM + max(MM_i)), i = 1{:}3\},$$

wherein PM is the probe intensity of the perfectly matched probe, which is designed to hybridize to the wild-type sequence for the base position; $MM_i$ is the probe intensity of one of the three mismatched probe, which is designed to hybridize to a single base pair substitution; and max(MM) is the maximum probe intensity among the three mismatched probes in the probe set.

13. The method of any one of claims 10 to 12, wherein the discrimination ability of the sense and anti-sense strand in step (c) is computed as a percentile of the values obtained in step (b) for probe sets in the strand at the base position measured using a plurality of microarrays.

14. A system for detecting a target nucleic acid in a test sample comprising:

a. a data acquisition module configured to acquire hybridization data from a microarray;
b. a data processing unit configured to process the data to determine a target nucleotide sequence by omitting a predetermined signal from one of the sense and anti-sense strands at one or more nucleotide positions in the target sequence determined by analysis of one or more training samples via the steps of: using one or more training samples and a plurality of microarrays comprising for each of said one or more nucleotide positions a sense and an antisense probe set specific for the sense or the anti-sense strand of the target nucleic acid, wherein each probe set contains five probes, four for each of the possible nucleotides at the particular position within the target sequence and one probe for a deletion of the nucleotide at that position, measuring hybridization signals at the nucleotide position using one or more probe sets for each of the sense and the anti-sense strands; for each probe set, determining base discrimination ability by comparing the hybridization signals within each probe set; for each nucleotide position, computing discrimination ability for sense and antisense strand separately using discrimination ability from each of the probe sets; for each nucleotide position, comparing discrimination ability between the sense and the anti-sense strands; in the test sample omitting the signal from a strand with lower base discrimination ability determined using the one or more training samples; and

c. a display module configured to display the data produced by the data processing unit.

15. The system of claim 14, wherein comparing the base discrimination between the probe sets corresponding to the sense and the anti-sense strands is performed by comparing a percentile of the base discrimination from sense and antisense strands obtained from the plurality of microarrays.

## Patentansprüche

1. Verfahren zum Abfragen einer Sequenz einer Zielnukleinsäure, die einen sense- und einen antisense-Strang aufweist, mittels einer Mikroarray-Untersuchung, welche einen Rechenvorgang zur Sequenzbestimmung umfasst, wobei im Rahmen dieses Verfahrens ein vorbestimmtes Signal vom Rechenvorgang ausgeschlossen wird, welches einem der sense- und antisense-Stränge zuzuordnen ist, wobei es eine oder mehrere Nukleotidpositionen in der Zielnukleinsäuresequenz betrifft, und welches durch Untersuchung eines oder mehrerer Übungsproben bestimmt wird, wobei das Ausschließen des vorbestimmten Signals aus einem der sense- und antisense-Stränge an einer Nukleotidposition die folgenden Schritte umfasst:

a. Verwenden einer oder mehrerer Übungsproben sowie mehrerer Mikroarrays, die für jede der Nukleotidpositionen, von denen eine oder mehrere vorliegen, einen Satz von sense- und antisense-Sonden umfasst, welche spezifisch für den sense- beziehungsweise antisense-Strang der Zielnukleinsäure sind, wobei jeder Sondensatz fünf Sonden enthält, vier für jedes der möglichen Nukleotide an der bestimmten Position innerhalb der Zielsequenz sowie eine Sonde für eine Deletion des Nukleotids an dieser Position, Messen von Hybridisierungssignalen an der Nukleotidposition unter Verwendung eines oder mehrerer Sondensätze für jeden der sense- und antisense-Stränge;

b. für jeden Sondensatz, Bestimmen des Vermögens zur Basendiskriminierung, indem die Hybridisierungssignale innerhalb jedes Sondensatzes verglichen werden;

c. für jede Nukleotidposition, gesondertes Berechnen des Diskriminierungsvermögens für den sense- bzw. den antisense-Strang, unter Verwendung des Diskriminierungsvermögens für jeden der Sondensätze, wie es in Schritt (b) bestimmt wurde;

d. für jede Nukleotidposition, Vergleichen des Diskriminierungsvermögens zwischen dem sense- und dem antisense-Strang, wie es in Schritt (c) berechnet wurde;

e. Ausschließen, bei der zu prüfenden Probe, des Signals, welches dem Strang mit geringerem Vermögen zur Basendiskriminierung zuzuordnen ist, wie es in Schritt (d) erkannt wurde, unter Verwendung der einen oder der mehreren Übungsproben.

2. Verfahren nach Anspruch 1, im Rahmen dessen weiterhin, in Schritt (d), bestimmt wird, ob der Unterschied hinsichtlich der Basendiskriminierung einen Schwellenwert überschreitet, und in Schritt (e) das Signal ausgeschlossen wird, das dem Strang zuzuordnen ist, welcher Sondensätzen mit geringerer Basendiskriminierung entspricht, sofern der Unterschied den Schwellenwert überschreitet.

3. Verfahren nach einem beliebigen der Ansprüche 1 oder 2, wobei die Sondenintensitäten in Schritt (a) unter Verwendung mehrerer Mikroarrays gemessen wird, die mit einer einzigen Probe in Kontakt gebracht werden.

4. Verfahren nach einem beliebigen der Ansprüche 1 oder 2, wobei das Hybridisierungssignal in Schritt (a) unter Verwendung mehrerer Mikroarrays gemessen wird, die mit mehreren Proben in Kontakt gebracht werden.

5. Verfahren nach einem beliebigen der Ansprüche 1 bis 4, wobei die Basendiskriminierung in Schritt (b) unter Verwendung der Formel 1 gemessen wird:

$$\text{DR\_MM}_s = \{(PM - \max(MM_i)) \ / \ (PM + \max(MM_i)), \ i = 1{:}3\},$$

wobei PM die Sondenintensität der uneingeschränkt gepaarten Sonde ist, welche dafür ausgelegt ist, an dieser Basenposition mit der Wildtypsequenz zu hybridisieren; $MM_i$ die Sondenintensität einer der drei fehlgepaarten Sonden ist, wobei diese dafür ausgelegt ist, mit einer Einzelbasenpaar-Substitution zu hybridisieren; und max (MM) die maximale Sondenintensität unter den drei fehlgepaarten Sonden aus dem Sondensatz ist.

6. Verfahren nach einem beliebigen der Ansprüche 1 bis 5, wobei das Diskriminierungsvermögen für den sense- und den antisense-Strang in Schritt (c) in Form eines Perzentils des Wertes berechnet wird, welcher in Schritt (b) für die Sondensätze in dem Strang an der Basenposition erzielt wurde.

7. Verfahren nach einem beliebigen der Ansprüche 1 bis 6, wobei in Schritt (d) das Diskriminationsvermögen zwischen sense und antisense, wie es in Schritt (c) berechnet wurde, unter Verwendung der Formel 3 verglichen wird:

$$(1) \qquad Q_{75i} < Q_{75j} - T, \qquad \text{für } Q_{75i} < PT$$

$$(2) \qquad Q_{75i} < A(Q_{75j} - B)^2 + PT, \qquad \text{für } Q_{75i} \geq PT$$

wobei T der Schwellenwert ist; $Q_{75i}$ der $Q_{75}$-Wert eines zu entfernenden Stranges i ist; und $Q_{75j}$ der $Q_{75}$-Wert des komplementären Stranges j ist.

8. Verfahren nach einem beliebigen der Ansprüche 6 oder 7, wobei das Perzentil zwischen 60 und 90 % liegt.

9. Verfahren nach einem beliebigen der Ansprüche 6 bis 8, wobei es sich bei dem Perzentil um das dritte Quartil (75 %) handelt.

10. Verfahren zum Nachweis des Vorhandenseins oder der Abwesenheit einer Zielnukleinsäure mit einem sense- und einem antisense-Strang in einer zu prüfenden Probe unter Verwendung einer Mikroarray-Untersuchung, welche einen Rechenvorgang zur Sequenzbestimmung oder zum Mutationsnachweis umfasst, wobei im Rahmen desselben ein vorbestimmtes Signal vom Rechenvorgang ausgeschlossen wird, welches einem der sense- und antisense-Stränge zuzuordnen ist, wobei es eine oder mehrere Nukleotidpositionen in der Zielnukleinsäuresequenz betrifft, und welches durch Untersuchung eines oder mehrerer Übungsproben bestimmt wird, wobei das Ausschließen des vorbestimmten Signals aus einem der sense- und antisense-Stränge an einer Nukleotidposition die folgenden Schritte umfasst:

a. Verwenden einer oder mehrerer Übungsproben sowie mehrerer Mikroarrays, die für jede der Nukleotidpositionen, von denen eine oder mehrere vorliegen, einen Satz von sense- und antisense-Sonden umfasst, welche spezifisch für den sense- beziehungsweise antisense-Strang der Zielnukleinsäure sind, wobei jeder Sondensatz fünf Sonden enthält, vier für jedes der möglichen Nukleotide an der bestimmten Position innerhalb der Zielsequenz sowie eine Sonde für eine Deletion des Nukleotids an dieser Position, Messen von Hybridisierungssignalen an der Nukleotidposition unter Verwendung eines oder mehrerer Sondensätze für jeden der sense- und antisense-Stränge;
b. für jeden Sondensatz, Bestimmen des Vermögens zur Basendiskriminierung, indem die Hybridisierungssignale innerhalb jedes Sondensatzes verglichen werden;
c. für jede Nukleotidposition, gesondertes Berechnen des Diskriminierungsvermögens für den sense- bzw. den antisense-Strang, unter Verwendung des Diskriminierungsvermögens für jeden der Sondensätze, wie es in Schritt (b) bestimmt wurde;
d. für jede Nukleotidposition, Vergleichen des Diskriminierungsvermögens zwischen dem sense- und dem antisense-Strang, wie es in Schritt (c) berechnet wurde;
e. Ausschließen, bei der zu prüfenden Probe, des Signals, welches einem Strang mit geringerem Vermögen zur Basendiskriminierung zuzuordnen ist, wie es in Schritt (d) erkannt wurde, unter Verwendung der einen oder der mehreren Übungsproben.

11. Verfahren nach Anspruch 10, im Rahmen dessen weiterhin, in Schritt (d), bestimmt wird, ob der Unterschied hinsichtlich der Basendiskriminierung einen Schwellenwert überschreitet, und, in Schritt (e), das Signal ausgeschlossen wird, das dem Strang zuzuordnen ist, welcher Sondensätzen mit geringerer Basendiskriminierung entspricht, sofern der Unterschied den Schwellenwert überschreitet.

12. Verfahren nach einem beliebigen der Ansprüche 10 oder 11, wobei das Vermögen zur Basendiskriminierung in Schritt (b) unter Verwendung der Formel 1 gemessen wird:

$$\mathrm{DR\_MM_s} = \{(\mathrm{PM} - \max(\mathrm{MM_i})) \;/\; (\mathrm{PM} + \max(\mathrm{MM_i})),\; i = 1{:}3\},$$

wobei PM die Sondenintensität der uneingeschränkt gepaarten Sonde ist, welche dafür ausgelegt ist, an dieser Basenposition mit der Wildtypsequenz zu hybridisieren; $MM_i$ die Sondenintensität einer der drei fehlgepaarten Sonden ist, wobei diese dafür ausgelegt ist, mit einer Einzelbasenpaar-Substitution zu hybridisieren; und max (MM) die maximale Sondenintensität unter den drei fehlgepaarten Sonden aus dem Sondensatz ist.

13. Verfahren nach einem beliebigen der Ansprüche 10 bis 12, wobei das Diskriminierungsvermögen für den sense- und den antisense-Strang in Schritt (c) in Form eines Perzentils des Wertes berechnet wird, welcher in Schritt (b) für die Sondensätze in dem Strang an der Basenposition erzielt wurde, wobei die Messung unter Verwendung mehrerer Mikroarrays erfolgte.

14. System zum Nachweis einer Zielnukleinsäure in einer zu prüfenden Probe, wobei es Folgendes umfasst:

    a. ein Datenerfassungsmodul, das dafür ausgelegt ist, Hybridisierungsdaten aus einem Mikroarray zu erfassen;
    b. eine Datenverarbeitungseinheit, die dafür ausgelegt ist, die Daten derart zu verarbeiten, dass eine Zielnukleotidsequenz bestimmt wird, indem ein vorbestimmtes Signal ausgeschlossen wird, welches einem der sense- und antisense-Stränge zuzuordnen ist, wobei es an einer oder mehreren Nukleotidpositionen in der Zielsequenz auftritt, und welches durch Untersuchung einer oder mehrerer Übungsproben gemäß den folgenden Schritten bestimmt wurde: Verwenden einer oder mehrerer Übungsproben sowie mehrerer Mikroarrays, die für jede der Nukleotidpositionen, von denen eine oder mehrere vorliegen, einen Satz von sense- und antisense-Sonden umfasst, welche spezifisch für den sense- beziehungsweise antisense-Strang der Zielnukleinsäure sind, wobei jeder Sondensatz fünf Sonden enthält, vier für jedes der möglichen Nukleotide an der bestimmten Position innerhalb der Zielsequenz sowie eine Sonde für eine Deletion des Nukleotids an dieser Position, Messen von Hybridisierungssignalen an der Nukleotidposition unter Verwendung eines oder mehrerer Sondensätze für jeden der sense- und antisense-Stränge; für jeden Sondensatz, Bestimmen des Vermögens zur Basendiskriminierung, indem die Hybridisierungssignale innerhalb jedes Sondensatzes verglichen werden; für jede Nukleotidposition, gesondertes Berechnen des Diskriminierungsvermögens für den sense- bzw. den antisense-Strang, unter Verwendung des Diskriminierungsvermögens für jeden der Sondensätze; für jede Nukleotidposition, Vergleichen des Diskriminierungsvermögens zwischen dem sense- und dem antisense-Strang; Ausschließen, bei der zu prüfenden Probe, des Signals, welches einem Strang mit geringerem Vermögen zur Basendiskriminierung zuzuordnen ist, wobei es unter Verwendung der einen oder der mehreren Übungsproben bestimmt wurde; und
    c. ein Anzeigemodul, das dafür ausgelegt ist, die Daten anzuzeigen, welche von der Datenverarbeitungseinheit ausgegeben wurden.

15. System nach Anspruch 14, wobei der Vergleich der Basendiskriminierung zwischen den Sondensätzen, welche dem sense- und dem antisense-Strang entsprechen, derart durchgeführt wird, dass die Perzentile der Basendiskriminierung, wie sie dem sense- bzw. dem antisense-Strang zuzuordnen sind und wie sie ausgehend von mehreren Mikroarrays erhalten wurden, verglichen werden.

**Revendications**

1. Procédé d'interrogation d'une séquence d'un acide nucléique cible ayant un brin sens et un brin anti-sens par une analyse par microréseaux comprenant un calcul de détermination de séquence, le procédé comprenant l'omission du calcul d'un signal prédéterminé provenant de l'un des brins sens et anti-sens pour une ou plusieurs positions de nucléotides dans la séquence d'acide nucléique cible déterminée par l'analyse d'un ou de plusieurs échantillons d'apprentissage, dans lequel l'omission du signal prédéterminé provenant de l'un des brins sens et anti-sens à une position de nucléotide comprend les étapes de :

a. utilisation d'un ou de plusieurs échantillons d'apprentissage et d'une pluralité de microréseaux, comprenant pour chacune desdites une ou plusieurs positions de nucléotides un ensemble de sondes sens et anti-sens spécifique du brin sens ou du brin anti-sens de l'acide nucléique cible, dans lequel chaque ensemble de sondes contient cinq sondes, quatre pour chacun des nucléotides possibles à la position particulière au sein de la séquence cible et une sonde pour une délétion du nucléotide à cette position, la mesure des signaux d'hybridation à la position de nucléotide à l'aide d'un ou de plusieurs ensembles de sondes pour chacun des brins sens et anti-sens ;

b. pour chaque ensemble de sondes, la détermination de la capacité de discrimination des bases par comparaison des signaux d'hybridation au sein de chaque ensemble de sondes ;

c. pour chaque position de nucléotide, le calcul de la capacité de discrimination pour le brin sens et le brin anti-sens séparément à l'aide de la capacité de discrimination de chacun des ensembles de sondes déterminée à l'étape (b) ;

d. pour chaque position de nucléotide, la comparaison de la capacité de discrimination entre les brins sens et anti-sens calculée à l'étape (c) ;

e. dans l'échantillon d'essai, l'omission du signal provenant du brin présentant une capacité de discrimination des bases inférieure identifié à l'étape (d) en utilisant le ou les échantillons d'apprentissage.

2. Procédé selon la revendication 1, comprenant en outre, à l'étape (d), la détermination si la différence dans la discrimination des bases se situe au-dessus d'une valeur seuil, et à l'étape (e), l'omission du signal provenant d'un brin correspondant aux ensembles de sondes présentant une discrimination des bases inférieure si la différence se situe au-dessus de la valeur seuil.

3. Procédé selon l'une quelconque des revendications 1 ou 2, dans lequel les intensités des sondes à l'étape (a) sont mesurées à l'aide d'une pluralité de microréseaux mis en contact avec un seul échantillon.

4. Procédé selon l'une quelconque des revendications 1 ou 2, dans lequel le signal d'hybridation à l'étape (a) est mesuré à l'aide d'une pluralité de microréseaux mis en contact avec une pluralité d'échantillons.

5. Procédé selon l'une quelconque des revendications 1 à 4, dans lequel la discrimination des bases à l'étape (b) est mesurée à l'aide de la formule 1 :

$$DR\_MM_s = \{(PM - max(MM_i)) / (PM + max(MM_i)), i = 1:3\},$$

dans laquelle PM est l'intensité de sonde de la sonde parfaitement appariée, qui est conçue pour s'hybrider à la séquence de type sauvage pour la position de base ; $MM_i$ est l'intensité de sonde de l'une des trois sondes non appariées, qui est conçue pour s'hybrider à une substitution de paire de bases simple ; et max(MM) est l'intensité de sonde maximale parmi les trois sondes non appariées dans l'ensemble de sondes.

6. Procédé selon l'une quelconque des revendications 1 à 5, dans lequel, à l'étape (c), la capacité de discrimination pour le brin sens et le brin antisens est calculée en percentile des valeurs obtenues à l'étape (b) pour les ensembles de sondes dans le brin à la position de base.

7. Procédé selon l'une quelconque des revendications 1 à 6, dans lequel à l'étape (d), la capacité de discrimination entre le sens et l'antisens calculée à l'étape (c) est comparée à l'aide de la formule 3 :

$$(1) \qquad Q_{75i} < Q_{75j} - T, \qquad\qquad pour\ Q_{75i} < PT$$

$$(2) \qquad Q_{75i} < A(Q_{75j} - B)^2 + PT, \qquad pour\ Q_{75i} \geq PT,$$

dans laquelle T est le seuil ; $Q_{75i}$ est la valeur $Q_{75}$ d'un brin i à éliminer ; et $Q_{75j}$ est la valeur $Q_{75}$ du brin complémentaire j.

**8.** Procédé selon l'une quelconque des revendications 6 ou 7, dans lequel le percentile est compris entre 60 et 90 %.

**9.** Procédé selon l'une quelconque des revendications 6 à 8, dans lequel le percentile est le troisième quartile (75 %).

**10.** Procédé de détection de la présence ou de l'absence d'un acide nucléique cible ayant un brin sens et un brin anti-sens dans un échantillon d'essai à l'aide d'une analyse par microréseau comprenant une détermination de séquence ou un calcul de détection de mutation, comprenant l'omission du calcul d'un signal prédéterminé provenant de l'un des brins sens et anti-sens pour une ou plusieurs positions de nucléotides dans la séquence d'acide nucléique cible déterminé par l'analyse d'un ou de plusieurs échantillons d'apprentissage, dans lequel l'omission du signal prédéterminé provenant de l'un des brins sens et anti-sens à une position de nucléotide comprend les étapes de :

a. utilisation d'un ou de plusieurs échantillons d'apprentissage et d'une pluralité de microréseaux, comprenant pour chacune desdites une ou plusieurs positions de nucléotides un ensemble de sondes sens et anti-sens spécifique du brin sens ou du brin anti-sens de l'acide nucléique cible, dans lequel chaque ensemble de sondes contient cinq sondes, quatre pour chacun des nucléotides possibles à la position particulière au sein de la séquence cible et une sonde pour une délétion du nucléotide à cette position, la mesure des signaux d'hybridation à la position de nucléotide à l'aide d'un ou de plusieurs ensembles de sondes pour chacun des brins sens et anti-sens ;
b. pour chaque ensemble de sondes, la détermination de la capacité de discrimination des bases par comparaison des signaux d'hybridation au sein de chaque ensemble de sondes ;
c. pour chaque position de nucléotide, le calcul de la capacité de discrimination pour le brin sens et le brin anti-sens séparément à l'aide de la capacité de discrimination de chacun des ensembles de sondes déterminée à l'étape (b) ;
d. pour chaque position de nucléotide, la comparaison de la capacité de discrimination entre les brins sens et anti-sens calculée à l'étape (c) ;
e. dans l'échantillon d'essai, l'omission du signal provenant d'un brin présentant une capacité de discrimination des bases inférieure identifié à l'étape (d) en utilisant le ou les échantillons d'apprentissage.

**11.** Procédé selon la revendication 10, comprenant en outre, à l'étape (d), la détermination si la différence de discrimination des bases se situe au-dessus d'une valeur seuil et à l'étape (e), l'omission du signal provenant d'un brin correspondant aux ensembles de sondes ayant une discrimination des bases inférieure si la différence se situe au-dessus de la valeur seuil.

**12.** Procédé selon l'une quelconque des revendications 10 ou 11, dans lequel la capacité de discrimination des bases à l'étape (b) est mesurée à l'aide de la formule 1 :

$$DR\_MM_s = \{(PM - \max(MM_i)) / (PM + \max(MM_i)), i = 1:3\},$$

dans laquelle PM est l'intensité de sonde de la sonde parfaitement appariée, qui est conçue pour s'hybrider à la séquence de type sauvage pour la position de base ; $MM_i$ est l'intensité de sonde de l'une des trois sondes non appariées, qui est conçue pour s'hybrider à une substitution de paire de bases simple ; et max(MM) est l'intensité de sonde maximale parmi les trois sondes non appariées dans l'ensemble de sondes.

**13.** Procédé selon l'une quelconque des revendications 10 à 12, dans lequel la capacité de discrimination du brin sens et du brin anti-sens à l'étape (c) est calculée en percentile des valeurs obtenues à l'étape (b) pour les ensembles de sondes dans le brin à la position de base mesurée à l'aide d'une pluralité de microréseaux.

**14.** Système de détection d'un acide nucléique cible dans un échantillon d'essai comprenant :

a. un module d'acquisition de données conçu pour acquérir des données d'hybridation à partir d'un microréseau ;
b. une unité de traitement de données conçue pour traiter les données afin de déterminer une séquence nucléotidique cible par l'omission d'un signal prédéterminé provenant de l'un des brins sens et anti-sens à une ou plusieurs positions de nucléotides dans la séquence cible déterminée par l'analyse d'un ou de plusieurs échantillons d'apprentissage par l'intermédiaire des étapes de : utilisation d'un ou de plusieurs échantillons d'apprentissage et d'une pluralité de microréseaux, comprenant pour chacune desdites une ou plusieurs positions de nucléotides un ensemble de sondes sens et anti-sens spécifique du brin sens ou du brin anti-sens de l'acide nucléique cible, dans lequel chaque ensemble de sonde contient cinq sondes, quatre pour chacun des nucléotides possibles à la position particulière au sein de la séquence cible et une sonde pour une délétion du nucléotide à cette position, la mesure des signaux d'hybridation à la position de nucléotide à l'aide d'un ou de plusieurs ensembles de sondes pour chacun des brins sens et anti-sens ; pour chaque ensemble de sondes, la détermination de la capacité de discrimination des bases par comparaison des signaux d'hybridation au sein de chaque ensemble de sondes ; pour chaque position de nucléotide, le calcul de la capacité de discrimination pour le brin sens et le brin anti-sens séparément à l'aide de la capacité de discrimination de chacun des ensembles de sondes ; pour chaque position de nucléotide, la comparaison de la capacité de discrimination entre les brins sens et anti-sens ; dans l'échantillon d'essai, l'omission du signal provenant d'un brin présentant une capacité de discrimination des bases inférieure déterminé en utilisant le ou les échantillons d'apprentissage ; et

c. un module d'affichage conçu pour afficher les données produites par l'unité de traitement de données.

15. Système selon la revendication 14, dans lequel la comparaison de la discrimination des bases entre les ensembles de sondes correspondant aux brins sens et anti-sens est réalisée par comparaison d'un percentile de la discrimination des bases des brins sens et anti-sens obtenu à partir de la pluralité de microréseaux.

FIGURE 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011040886 A1

### Non-patent literature cited in the description

- **SCHWARTZ, S.** Clinical Utility of Single Nucleotide Polymorphism Arrays. *Clin. Lab. Med.,* 2011, vol. 31, 581
- **ZIN R. et al.** SNP-based arrays complement classic cytogenetics in the detection of chromosomal aberrations in Wilms' tumor. *Cancer Genetics,* 2012, vol. 205, 80
- **LEE C.W.H. et al.** Large-scale evolutionary surveillance of the 2009 H1N1 influenza A virus using resequencing arrays. *Nucleic Acids Research,* 2010, vol. 38 (9), 1-14
- **ZHAN Y. ; KULP D.** Model-P: A basecalling method for resqeuencing microarrays of diploid samples. *Bioinformatics,* 2005, vol. 21 (2), ii182-ii189
- Microarray Biochip Technology. Eaton Pub. Co, 2000
- Microarray Biochip Technology. 2000
- **SEO.** *Bioinformatics,* 2004, vol. 20 (16), 2534-2544
- **FREED-PASTOR, W. et al.** Mutant p53: one name, many proteins. *Genes Dev.,* 2004, vol. 26, 1268